(19) Europäisches Patentamt
European Patent Office
Office européen des brevets

(11) Publication number: **0 258 655 B1**

(12) # EUROPEAN PATENT SPECIFICATION

(45) Date of publication of patent specification: **06.11.91** (51) Int. Cl.$^5$: **C07F 15/00**

(21) Application number: **87111190.2**

(22) Date of filing: **03.08.87**

(54) **Complexes of platinum (II) 2,3-dinitrilo-2-butene-2,3-dithiolate, a process for preparing them and pharmaceutical compositions containing them.**

(30) Priority: **04.08.86 ES 8601525**

(43) Date of publication of application:
**09.03.88 Bulletin 88/10**

(45) Publication of the grant of the patent:
**06.11.91 Bulletin 91/45**

(84) Designated Contracting States:
**BE CH DE FR GB IT LI NL**

(56) References cited:
**EP-A- 0 215 393**

**Journal of the American Chemical Society, vol. 105, no.7, April-May 1983, pages 1795-1802, American Chemical Society, Washington, D.C., US; C.E. Johnson et al.: "Luminiscent Iridium(I), Rhodium(I), and Platinum(II); Dithiola te Complexes" page 1797,column 2**

(73) Proprietor: **FERRER INTERNACIONAL, S.A.**
**Gran Via Carlos III, 94**
**08028 Barcelona(ES)**

(72) Inventor: **Foguet, Rafael**
**Lusanée 8**
**ES-08022 Barcelona(ES)**
Inventor: **Sampedro, Frederico**
**Francia 18-20**
**ES-08024 Barcelona(ES)**
Inventor: **Ortiz, José A.**
**Corcega 429**
**ES-08037 Barcelona(ES)**
Inventor: **Cayuela, Salvador**
**Loreto 46**
**ES-08029 Barcelona(ES)**
Inventor: **Castello, Josep M.**
**Princep d'Asturias 35**
**ES-08012 Barcelona(ES)**
Inventor: **De Andrés, Luis**
**Tarragona 40**
**El Papiol Barcelona(ES)**
Inventor: **Bonal, Joaquin**
**Reus 26**
**ES-08022 Barcelona(ES)**

(74) Representative: **Kinzebach, Werner, Dr.**
**Patentanwälte Reitstötter, Kinzebach und Partner Sternwartstrasse 4 Postfach 86 06 49**
**W-8000 München 86(DE)**

**EP 0 258 655 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to platinum(II) complexes of 2,3-dimercapto-2-butene-dinitrilate(2-)-S,S' derivatives of the general formula (I):

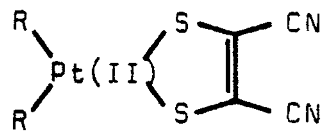

(I)

wherein R is a dialkylsulphide or trialkylphosphine group and each alkyl group may contain one or more (two) hydroxyl groups and has 1 to 2 carbon atoms, as well as to a process for preparing the same and to pharmaceutical compositions containing these compounds.

The compounds of the present invention are prepared, in accordance with the below scheme, from the respective cis-dichloro platinum(II) bis-substituted intermediates of the general formula (II):

cis-$[Pt^{(II)}Cl_2\{R\}_2]$ (II)

wherein R is as defined for (I), and from the metallic cis-1,2-dicyano-1,2-ethylenedithiolate of general formula (III):

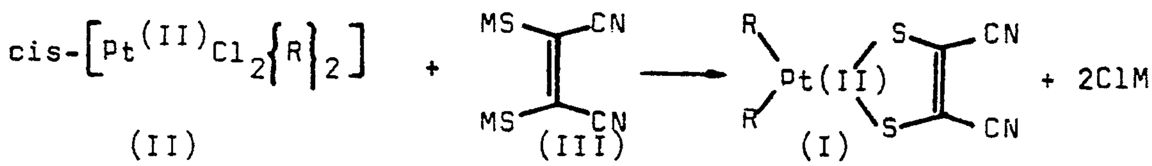

(II) (III) (I)

wherein M is a monovalent metal, preferably an alkali metal, in particular sodium.

The above reactions occur suitably at room temperature, and a ketone or an alcohol having 1 to 4 carbon atoms may be used as solvents; acetone and methanol are preferred, although other ketones or alcohols having 1 to 4 four carbon atoms are also acceptable for this reaction, for example methyl ethyl ketone, ethanol, n-propanol or i-propanol. The end products thus obtained are purified by crystallization or simply by washing the formed precipitate.

Respective cis-dichloro platinum(II) bis-substituted precursors of the general formula (II) are obtained by conventional methods when R is a dialkylsulphide or trialkylphosphine group without hydroxyl groups (Kauffman,GB and Cowan, DO: Inorg.Synth.(1963),7,239-245; KauffmanGB and Cowan,DO: Ibid (1960)-,6,211-215; Parshall,GW: Ibid (1970),12,26-33) according to the following reactions:

$$Pt \xrightarrow[NO_3H]{HCl} H_2PtCl_6 + \text{nitrcus gases}$$

$$H_2PtCl_6 + KCl \longrightarrow K_2PtCl_6 + HCl$$

$$K_2PtCl_6 + C_2O_4K_2 \longrightarrow K_2PtCl_4 + 2CO_2 + 2KCl$$

$$K_2PtCl_4 + 2(H_5C_2)_2S \longrightarrow \text{trans-}[PtCl_2\{(H_5C_2)_2S\}_2] + 2KCl$$

$$\text{trans-}[PtCl_2\{(H_5C_2)_2S\}_2] + 2(H_5C_2)_2S \longrightarrow [Pt\{(H_5C_2)_2S\}_4]Cl_2$$

$$[Pt\{(H_5C_2)_2S\}_4]Cl_2 \longrightarrow \text{cis-}[PtCl_2\{(H_5C_2)_2S\}_2] + 2(H_5C_2)_2S$$

$$K_2PtCl_4 + 2(H_5C_2)_3P \longrightarrow \text{cis-}[PtCl_2\{(H_5C_2)_3P\}_2] + 2KCl$$

When R is a trialkylphosphine group with hydroxyl groups, the corresponding cis-dichloro platinum(II)

bis-substituted precursor may be advantageously obtained by reacting tris-hydroxyalkylphosphine with dichloro-1,5-cyclooctadiene platinum (Spanish Patent of Addition No. 548,849). Metallic cis-1,2-dicyano-1,2-ethylenedithiolates of general formula (III) are obtained from the corresponding cyanide.

Thus, sodium cis-1,2-dicyano-1,2-ethylenedithiolate (III, M = Na) is also obtained by conventional methods: sodium cyanide is reacted with carbon disulphide and N,N-dimethylformamide (Bahr,G and Schleitzer,G: Chem.Ber.(1957),90,438) according to the following reaction:

$$NaCN + CS_2 + 3HCON(CH_3)_2 \longrightarrow \underset{(monomer)}{NCCS_2Na.3HCON(CH_3)_2}$$

followed by dimerization (Locke,J and McCleverty,JA: Inorg. Chem.(1966),5,1157) according to the following reaction:

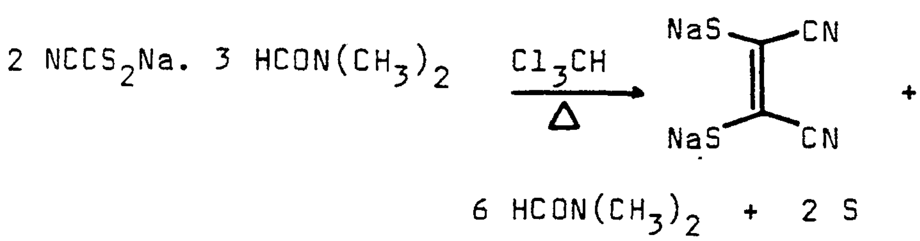


The compounds of the present invention are useful as a medication in the treatment of tumors and may be administered, mixed with suitable carriers, orally in the form of tablets, capsules, coated-tablets, granules, syrup, solution, etc., or by injection, at daily doses ranging from 10 to 600 mg/m$^2$.

A number of examples will now be described in non-limitative manner to illustrate the invention.

## Example 1

Platinum(II)[2,3-dimercapto-2-butene-dinitrilate (2-)-S,S']bis(dietylsulphide)

A mixture of 0.5 g (1.12 mmole) of cis-[Pt$^{(II)}$Cl$_2${S(CH$_2$CH$_3$)$_2$}$_2$] and 0.2005 g (1.12 mmole) of sodium cis-1,2-dicyano-1,2-ethylenedithiolate was dissolved in 100 ml of acetone, then the yellow-coloured solution changed to orange. The solution was filtered off and the liquid was concentrated to give reddish-orange crystals which were recrystallized from acetone.

Yield: 0.31 g (53.7%).

Melting point: 141-145° C.

IR Spectrum (KBr) cm$^{-1}$: 2960, 2930, 2865, 2205, 1495, 1440, 1430, 1370, 1275, 1250, 1152, 1105, 1075, 1045, 970, 780, 510.

NMR Spectrum (CD$_3$COCD$_3$)$\delta$: 3.4-1.75 (m), 1.5-1.2 (t).

## Example 2

Platinum(II)[2,3-dimercapto-2-butene-dinitrilate (2-)-S,S']bis(triethylphosphine)

0.1853 g (1.0 mmole) of sodium cis-1,2-dicyano-1,2-ethylenedithiolate were dissolved in 100 ml of methanol and 0.5 g (1.0 mmole) of cis-[Pt$^{(II)}$Cl$_2${P(CH$_2$CH$_3$)$_3$}$_2$] were added. The yellowish solution faded and turned turbid. The solution was filtered off and the precipitate washed with methanol, and then dried. 0.3 g of pale-red product was obtained.

Yield: 54%.

Melting point: 230-232° C.

IR Spectrum (KBr) cm$^{-1}$: 2970, 2930, 2870, 2200, 1496, 1450, 1415, 1380, 1250, 1150, 1038, 765, 728, 638, 515.

NMR Spectrum (CD$_3$CCCD$_3$) $\delta$ : 2.6-1.9 (m), 1.4-0.9 (m).

Example 3

Platinum(II)[2,3-dimercapto-2-butene-dinitrilate (2-)-S,S']bis(tris-hydroxymethylphosphine)

0.3 g (1.16 mmole) of sodium cis-1,2-dicyano-1,2-ethylenedithiolate were dissolved in 100 ml of methanol and 0.83 g (1.16 mmole) of cis-$[Pt^{(II)}Cl_2\{P(CH_2OH)_3\}_2]$ were added. The yellow-coloured solution changed to orange. The solution was concentrated and left to crystallize. The solid obtained was dissolved in acetone. The soluble phase in acetone was dried and recrystallized from methanol to give dark-red crystals.

Yield: 0.81 g (86%).
Melting point: 203-206 °C.
IR Spectrum (KBr) $cm^{-1}$ : 3600-2900, 2220, 1690, 1505, 1438, 1160, 1035, 885, 840.
NMR ($CD_3OD$) δ : 4.9-4.3 (t) J = 24.4Hz.

## Claims

1. Platinum(II) complexes of 2,3-dimercapto-2-butene-dinitrilate(2-)-S,S' derivatives of the general formula (I):

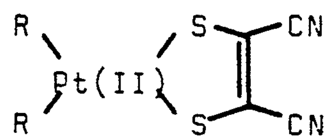

(I)

wherein R is a dialkylsulphide or trialkylphosphine group and each alkyl group may contain one or more hydroxyl groups and has 1 to 2 carbon atoms.

2. A process for preparing the compounds according to Claim 1, characterized in that the compounds of general formula (II):

cis-$[Pt^{(II)}Cl_2\{R\}_2]$ (II)

wherein R is as defined for (I), are reacted with a compound of general formula (III):

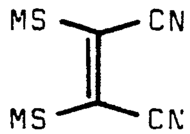

(III)

wherein M is a monovalent metal, and a ketone or an alcohol having 1 to 4 carbon atoms may be used as solvents.

3. The process of Claim 2, characterized in that M is sodium.

4. The process fo Claim 2, characterized in that the ketone used as solvent is acetone.

5. The process of Claim 2, characterized in that the alcohol used as solvent is methanol.

6. Pharmaceutical compositions comprising at least one of the compounds of Claim 1, optionally together with pharmaceutical carriers and/or adjuvants.

7. The use of the compounds according to Claim 1 for the preparation of pharmaceutical compositions for treating tumor diseases.

## Revendications

**1.** Complexes de platine (II) de dérivés 2,3-dimercapto-2-butène-dinitrilate(2-)-S,S' de la formule générale (I) :

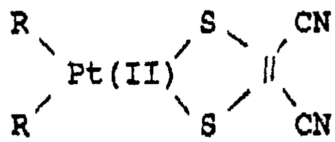

(I)

où R est un groupe dialkylsulfure ou trialkylphosphine et chaque groupe alkyle peut renfermer un ou plusieurs groupes hydroxyle et il a 1 à 2 atomes de carbone.

**2.** Procédé pour préparer des composés selon la revendication 1, caractérisé en ce que l'on fait réagir les composés de la formule générale (II)

$Cis\text{-}[Pt^{(II)}Cl_2\{R\}_2]$ (II)

où R est comme défini pour (I), avec un composé de la formule générale (III) :

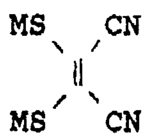

(III)

où M est un métal monovalent, et une cétone ou un alcool ayant de 1 à 4 atomes de carbone peut être utilisé comme solvant.

**3.** Procédé selon la revendication 2, caractérisé en ce que M est le sodium.

**4.** Procédé selon la revendication 2, caractérisé en ce que la cétone utilisée comme solvant est l'acétone.

**5.** Procédé selon la revendication 2, caractérisé en ce que l'alcool utilisé comme solvant est le méthanol.

**6.** Composition pharmaceutique comprenant au moins un des composés de la revendication 1, le cas échéant en combinaison avec des vecteurs et/ou des adjuvants pharmaceutiques.

**7.** Utilisation de composés selon la revendication 1 pour la préparation de compositions pharmaceutiques pour le traitement de maladies tumorales.

**Patentansprüche**

**1.** Platin-(II)-Komplexe von 2,3-Dimercapto-2-buten-dinitrilat(2-)-S,S'-Derivaten der allgemeinen Formel (I):

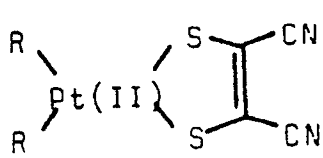

(I)

worin R eine Dialkylsulfid- oder Trialkylphosphingruppe bedeutet und jede Alkylgruppe ein oder mehrere Hydroxygruppen enthalten kann und 1 bis 2 Kohlenstoffatome aufweist.

**2.** Verfahren zur Herstellung der Verbindungen nach Anspruch 1, dadurch gekennzeichnet, daß man die

Verbindungen der allgemeinen Formel (II):

cis-[$Pt^{(II)}Cl_2\{R\}_2$]  (II)

worin R die für (I) angegebenen Bedeutungen besitzt, mit einer Verbindung der allgemeinen Formel (III):

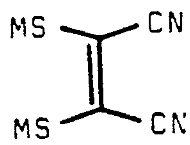

(III)

worin M für ein einwertiges Metall steht, umsetzt, wobei man ein Keton oder einen Alkohol mit 1 bis 4 Kohlenstoffatomen als Lösungsmittel verwenden kann.

3. Verfahren nach Anspruch 2, dadurch gekennzeichnet, daß M für Natrium steht.

4. Verfahren nach Anspruch 2, dadurch gekennzeichnet, daß das als Lösungsmittel verwendete Keton Aceton ist.

5. Verfahren nach Anspruch 2, dadurch gekennzeichnet, daß der als Lösungsmittel verwendete Alkohol Methanol ist.

6. Pharmazeutische Mittel, enthaltend wenigstens eine der Verbindungen nach Anspruch 1, gegebenenfalls zusammen mit pharmazeutischen Trägern und/oder Adjuvantien.

7. Verwendung der Verbindungen nach Anspruch 1 zur Herstellung pharmazeutischer Mittel zur Behandlung von Tumorerkrankungen.